# EUROPEAN PATENT APPLICATION

(11) **EP 4 521 340 A1**
(43) Date of publication of application: **12.03.2025**
(21) Application number: 23195974.3
(22) Date of filing: 07.09.2023
(51) Int. Cl.: G06T 7/11, G06T 19/00, G16H 30/40, G06N 3/09, G06N 3/0895

(54) **TRAINING OF IMAGE PROCESSING NEURAL NETWORKS TO SEGMENT THREEDIMENSIONAL MEDICAL IMAGES**

(71) Applicant: Koninklijke Philips N.V., 5656 AG Eindhoven (NL)
(72) Inventor: BROSCH, Tom, Eindhoven (NL); FLÄSCHNER, Nick, Eindhoven (NL); EWALD, Arne, 5656AG Eindhoven (NL); SCHMIDT-RICHBERG, Alexander, Eindhoven (NL); CAROLUS, Heike, Eindhoven (NL); GESSERT, Nils Thorben, Eindhoven (NL); LOSSAU, Tanja, Eindhoven (NL)
(74) Representative: Philips Intellectual Property & Standards

(57) **Abstract**

Disclosed herein is a medical imaging method. The method comprises receiving an image processing neural network (122) configured to output a segmentation. The method further comprises receiving a training three-dimensional medical image (123). The method comprises repeatedly: rendering a cross sectional view (124) of the training three-dimensional medical image; receiving segmentation edit data (129) from a segmentation entry tool (128); constructing a manual segmentation (132) using the segmentation edit data; and collecting per-voxel tool use metadata descriptive of use of the segmentation entry tool. The method further comprises: at least partially determining a per-voxel confidence score (138) using the per-voxel tool use metadata; constructing training data (140); and training the image processing neural network using the training data. The training of the image processing neural network is adjusted using the per-voxel confidence score.

## Description

### FIELD OF THE INVENTION

The invention relates to medical imaging, in particular to the segmentation of medical images.

### BACKGROUND OF THE INVENTION

There exist various imaging modalities which enable the acquisition of three-dimensional medical images which are descriptive of the internal anatomy of a subject. For example, a subject can be imaged using magnetic resonance imaging, computed tomography, positron emission tomography, and single photon emission tomography. Often times three-dimensional medical images are segmented. In segmentation, various regions of normal and/or abnormal are identified and delineated. Segmentation may be very useful for such things as tracking the growth of tumors over time or used during radiation therapy planning to control the amount of radiation delivered to different organs.

United States patent application US2016364631A1 discloses a system for automatically analyzing clinical images using rules and image analytics. One system includes a server including an electronic processor and an interface for communicating with at least one data source. The electronic processor is configured to receive training information from the at least one data source over the interface. The training information includes a plurality of images and graphical reporting associated with each of the plurality of images. The electronic processor is also configured to perform machine learning to develop a model using the training information and receive an image for analysis. The electronic processor is also configured to determine a set of rules for the image and automatically process the image using the model and the set of rules to generate a diagnosis for the image.

### SUMMARY OF THE INVENTION

The invention provides for a medical system, a computer program, and a method in the independent claims. Embodiments are given in the dependent claims.

In one aspect the invention provides for a medical system that comprises a memory storing machine-executable instructions. The medical system further comprises a user interface. The medical system further comprises a computational system. Execution of the machine-executable instructions causes the computational system to receive an image processing neural network. The image processing neural network is configured to output a segmentation of at least a portion of a clinical three-dimensional medical image in response to receiving at least a portion of the clinical three-dimensional medical image as input.

Execution of the machine-executable instructions further causes the computational system to receive a training three-dimensional medical image. The training three-dimensional medical image comprises voxels.

Execution of the machine-executable instructions further causes the computational system to repeatedly render a cross-sectional view of the training three-dimensional medical image on a user interface. The user interface comprises a segmentation entry tool. The segmentation entry tool as used herein may include a tool or user interface object or control which enables the entry of data in order to modify or input segmentation edit data. Execution of the machine-executable instructions further causes the computational system to repeatedly receive segmentation edit data from the segmentation entry tool. Execution of the machine-executable instructions further causes the computational system to repeatedly construct a manual segmentation using the segmentation edit data. Execution of the machine-executable instructions further causes the computational system to repeatedly collect per-voxel tool use metadata that is descriptive of the use of the segmentation entry tool to enter the segmentation data for voxels within a predefined distance of a location of the segmentation entry tool when the segmentation edit data is received.

Execution of the machine-executable instructions further causes the computational system to at least partially determine a pre-voxel confidence score using the per-voxel tool use metadata. Execution of the machine-executable instructions further causes the computational system to construct training data using the training three-dimensional medical image as input data and the manual segmentation as output data. The input data and the output data form a pair of data for training data. Execution of the machine-executable instructions further causes the computational system to train the image processing neural network using the training data. It should be noted that this process during the training process may possibly be repeated many times. The training of the image processing data is adjusted using the per-voxel confidence score.

In another aspect, the invention provides for a medical imaging method. The method comprises receiving an image processing neural network. The image processing neural network is configured to output a segmentation of at least a portion of a clinical three-dimensional medical image in response to receiving the at least a portion of a clinical three-dimensional medical image as input. The method further comprises receiving a training three-dimensional medical image. The training three-dimensional medical image comprises voxels.

The method comprises repeatedly rendering a cross-sectional view of the training three-dimensional medical image on a user interface. The user interface comprises a segmentation entry tool. The method further comprises repeatedly receiving segmentation edit data from the segmentation entry tool. The method further comprises repeatedly constructing a manual segmentation using the segmentation edit data. The method further comprises repeatedly collecting per-voxel tool use metadata that is descriptive of the use of the segmentation entry tool to enter the segmentation data for voxels within a predefined distance of a location of the segmentation entry tool when the segmentation edit data is received.

The method further comprises at least partially determining a per-voxel confidence score using the per-voxel tool use metadata. The method further comprises constructing training data using the training three-dimensional medical image as input data and the manual segmentation as output data. The method further comprises training the image processing neural network using the training data. The training of the image processing neural network is adjusted using the per-voxel confidence score.

In another aspect the invention provides for a computer program that comprises machine-executable instructions configured for execution by a computational system that controls a medical system. The medical system comprises a user interface. Execution of the machine-executable instructions causes the computational system to receive an image processing neural network. The image processing neural network is configured to output a segmentation of at least a portion of a clinical three-dimensional medical image in response to receiving the at least a portion of a clinical three-dimensional medical image as input. Execution of the machine-executable instructions further causes the computational system to receive a training three-dimensional medical image. The training three-dimensional medical image comprises voxels.

Execution of the machine-executable instructions further causes the computational system to repeatedly render a cross-sectional view of the training three-dimensional medical image on a user interface. The user interface comprises a segmentation entry tool. Execution of the machine-executable instructions further causes the computational system to repeatedly receive segmentation edit data from the segmentation entry tool. Execution of the machine-executable instructions further causes the computational system to repeatedly construct a manual segmentation using the segmentation edit data. Execution of the machine-executable instructions further causes the computational system to repeatedly collect per-voxel tool use metadata that is descriptive of the use of a segmentation entry tool to enter the segmentation data for voxels within a predefined distance of a location of the segmentation entry tool when the segmentation edit data is received.

Execution of the machine-executable instructions further causes the computational system to at least partially determine a per-voxel confidence score using the per-voxel tool use metadata. Execution of the machine-executable instructions further causes the computational system to construct training data using the training three-dimensional medical image as input data and the manual segmentation as output data. Execution of the machine-executable instructions further causes the computational system to train the image processing neural network using the training data. The training of the image processing neural network is adjusted using the per-voxel confidence score.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a medical system.
Fig. 4 shows a flow chart which illustrates a method of using the medical system of Fig. 3.
Fig. 5 illustrates an example of an imaging processing neural network.
Fig. 6 illustrates a further example of an image processing neural network.
Fig. 7 illustrates an example of a trained machine learning module.
Fig. 8 illustrates a further example of a trained machine learning module.

### DESCRIPTION OF EMBODIMENTS

Like numbered elements in these figures are either equivalent elements or perform the same function. Elements which have been discussed previously will not necessarily be discussed in later figures if the function is equivalent.

The manual delineation and annotation of anatomical and pathological structures in three-dimensional medical images during a clinical routine are often driven by a clinical question, which also influences the diligence with which the task is performed, resulting in highly accurate annotation of structures relevant to the clinical question, but also significantly less accurate segmentation of structures, where high fidelity is not required to answer the clinical question at hand. This renders large amounts of annotations collected in clinical routine unusable for training of AI-based algorithms such as image processing neural networks, because training may require consistently accurate annotations.

Examples may provide for a means of enabling the use of segmentations that were previously not suitable for training AI-based algorithms. Examples may overcome this problem by estimating local annotation confidence scores based on the input behavior of the annotating radiologist. This local confidence score may be referred to as a the per-voxel confidence score. The per-voxel confidence score may be used to distinguish between regions of high annotation quality, which are suitable for training, and regions that should be disregarded during training. Thereby, also annotations that only partially meet the quality requirements for training AI-based algorithms may possibly be leveraged to train more accurate and robust algorithms.

The user interface may include such things as a display and various types of human interface devices such as a mouse, a keyboard, a graphical pen system or other device which enables an operator to enter data into or manipulate a user interface. The computational system may include one or more computational systems located at one or more locations. The image processing neural network may encompass a neural network which is able to receive an image as input and to also output image data.

The segmentation output by the image processing neural network may be in the form of an image that is output or the output may be a standardized mesh. For example, when segmenting a clinical three-dimensional medical image there are several different ways of specifying the location of the segmentation. One way would be to mark the segmentation by delineating it using various voxels. Another way to do this would be to define the location of vertices for a three-dimensional mesh by providing coordinates for the different vertices. In one case, the segmentation is specified by indicating the location of the segmentation by marking particular voxels as containing the segmentation. In other cases the output of the image processing neural network can be configured such that instead of outputting the position of individual voxels it outputs the coordinates of the vertices of the three-dimensional mesh.

The training three-dimensional medical image and the other medical images which may input into the image processing neural network may be either three-dimensional datasets or they may be stacks of two-dimensional images.

The predefined distance may for example be determined in terms of a distance if the scale of the images used or it may be within a certain number of voxels or within a surrounding number of layers for a three-dimensional dataset.

As was mentioned above, execution of the machine-executable instructions further causes the computational system to at least partially determine a pre-voxel confidence score using the per-voxel tool use metadata. This may be accomplished in several different ways. In one case per-voxel tool use metadata could be collected and then used to calculate the per-voxel confidence score using an equation. In other examples, a trained machine learning module could be configured to output a score.

The training of the image processing data may be adjusted using the per-voxel confidence score. This means that the training of the image processing neural network is modified using the per-voxel confidence score. This again may take different forms in different examples. In one case the per-voxel confidence score could for example be used to modify or weight a loss function used in deep learning training. In other instances, the per-voxel confidence score is used to exclude or emphasize some data during the training process. For example, if portions of the segmentation of the training three-dimensional medical image were performed less rigorously this may be reflected in the per-voxel confidence score and the region with the less detailed or less accurate segmentation would be de-emphasized or not used.

In another example, the per-voxel tool use metadata comprises a zoom factor of the rendering of the cross-sectional view during entry of the segmentation edit data. In this example, the degree to which the display of the cross-sectional view if zoomed may be indicative of how accurately or how careful the segmentation edit data was entered. For example, if the zoom factor is greatly zoomed out, this may indicate that the segmentation edit data was entered with less care. In contrast, if the zoom factor were increased such that the cross-sectional view showed a smaller area and/or was greatly magnified, then this would ease the ability to enter the segmentation edit data more accurately. The per-voxel tool use metadata in this example may comprise a numerical value which is used to represent the zoom factor. This numerical value for the zoom factor could for example be put into an equation or a weighted average to numerically calculate the per-voxel confidence score. In other examples the per-voxel confidence score is calculated using a trained machine learning module. In this case the zoom factor could be encoded for entry into the training machine learning module. For example, a numerical value representing the zoom factor could be input into the training machine learning module.

In another example, the per-voxel tool use metadata comprises a description if the per-voxel tool use metadata for a voxel was rendered in the user interface when the segmentation edit data was entered. In this example, the user interface may provide feedback to the user to indicate the value of the per-voxel tool use metadata as the segmentation edit data is being entered. If this data is being disclosed to the user of the user interface then this may be used to adjust the value of the per-voxel confidence score.

In another example, the per-voxel tool use metadata comprises a segmentation entry tool type. This may be significant because different types of tools for entering the segmentation edit data may have an effect on how accurately or how precisely the data was entered. This may also include such things as properties about having a larger or smaller tool, which would affect how accurately the segmentation edit data can be entered. The choice of the segmentation entry tool type may be encoded for use in an equation or weighted average. It may also be encoded numerically for input into a trained machine learning module which is configured to output the per-voxel confidence score.

In another example, the per-voxel tool use metadata comprises a segmentation entry tool size. This may be beneficial because the segmentation entry tool size may affect how accurately or how much care was put into entering the segmentation edit data. The segmentation entry tool size may also be encoded numerically for entry into an equation or for entry into the above mentioned training machine learning module.

In another embodiment the voxel tool use metadata comprises a ball size. For example, the segmentation entry tool may be a ball which is used to push or distort a three-dimensional mesh. The ball size then has a great effect on how accurately or how large of an area is affected during an edit procedure. The ball size may be encoded numerically for use in an equation or weighted average for calculating the score or it may also be encoded numerically for input into the trained machine learning module.

In another example the per-voxel tool use metadata comprises a live-wire diameter.

In another example the per-voxel tool use metadata comprises a point annotation size.

In another example the per-voxel tool use metadata comprises an interactive region growing size.

As mentioned above, the live-wire diameter, the point annotation size, and the interactive region growing size may be numerically encoded for inclusion into an equation used to calculate the score or for input into the trained machine learning module.

In another example, the per-voxel tool use metadata comprises a maximum segmentation entry tool velocity. The maximum segmentation entry tool velocity may indicate how quickly the segmentation entry tool was moved during the entry of the segmentation entry data. Giving a higher maximum velocity may indicate that less care was taken when entering the segmentation edit data. Numerical values can be used to encode the maximum segmentation entry tool velocity for inclusion in an equation or for entry into the trained machine learning module.

The per-voxel tool use metadata further comprises a minimum segmentation entry tool velocity. The per-voxel tool use metadata further comprises an average segmentation entry tool velocity.

The minimum segmentation entry tool velocity and the average segmentation entry tool velocity may be also encoded numerically for use in an equation to calculate the score as well as for entry into the trained machine learning module.

In another example the per-voxel tool use metadata comprises a flag indicating eraser tool use within the predefined distance.

In another embodiment the per-voxel tool use metadata comprises a counter indicating a number of revisions to the manual segmentation within the predefined distance.

In both the example with the flag indicating the eraser tool use and the counter indicating a number of revisions the per-voxel tool use metadata can be encoded numerically, either for use in an equation to calculate the score numerically or to encode it for input into a trained machine learning module.

In another example, the per-voxel confidence score is a metric calculated using the per-voxel tool use metadata. A metric, as used herein, is an equation which may be used to calculate the per-voxel confidence score.

In another example, the memory further stores a trained machine learning module that is configured to output a machine-generated confidence score for voxels in response to receiving the per-voxel tool use metadata as input. Execution of the machine-executable instructions further comprises receiving the machine-generate confidence score for the voxels in response to inputting the per-voxel tool use metadata as input into the trained machine learning module.

The trained machine learning module may be implemented using different techniques. For example, it could be a linear regression module, a support vector machine, a random forest, implemented as a neural network. Examples of neural networks which may work would be a U-Net, an F-Net, a convolutional neural network, and a neural network which comprises fully connected layers. The trained machine learning module may be trained by constructing training data from examples of per-voxel tool use metadata which has been scored by an individual or labeled.

In another example the trained machine learning module is further configured to receive the three-dimensional medical image and the manual segmentation as input with the per-voxel tool use metadata. Execution of the machine-executable instructions further causes the computational system to input the three-dimensional medical image and the manual segmentation as input with the per-voxel tool use metadata into the trained machine learning module. In addition to receiving the per-voxel tool use metadata the image or a portion of the three-dimensional medical image and its associated manual segmentation are also input. The trained machine learning module such as a neural network and in particular a U-Net may be trained to recognize the level of detail in an image and compare it to the associated segmentation. This may be beneficial because it may provide a much improved rating or determination of the per-voxel confidence score.

In another example the image processing neural network is trained using a loss function. The training of the image processing data is adjusted using the per-voxel confidence score by weighting a contribution by individual voxels to the loss function using the per-voxel confidence score. For example, the per-voxel confidence score could be multiplied by the contribution to the loss function by a particular voxel.

In another example, the image processing neural network is configured to receive only a windowed portion of the training three-dimensional image. The training of the image processing neural network is adjusted using the per-voxel confidence score by restricting the windowed portion of the training three-dimensional image to regions where the per-voxel confidence score is above a predetermined threshold.

It is possible that the image processing neural network is configured to receive an image which is smaller than the training three-dimensional image. In which case, the image processing neural network would be configured to output just a portion of the segmentation. This can be used in the above example by selecting portions of the training three-dimensional image which have a better segmentation. Portions of the segmentation which are rated as being worse according to the per-voxel confidence score may have them be de-emphasized by either not using them for the training of the image processing neural network or reducing their significance in the training.

In another example, execution of the machine-executable instructions further causes the computational system to receive a clinical three-dimensional medical image. Execution of the machine-executable instructions further causes the computational system to receive a segmentation of the clinical three-dimensional medical image in response to inputting the clinical three-dimensional medical image into the image processing neural network. In this example the image processing neural network which has been trained is then used to generate a segmentation of the clinical three-dimensional medical image. This embodiment may be beneficial because the use of the per-voxel confidence score may have provided for a better trained and thereby a more accurate segmentation of the clinical three-dimensional medical image.

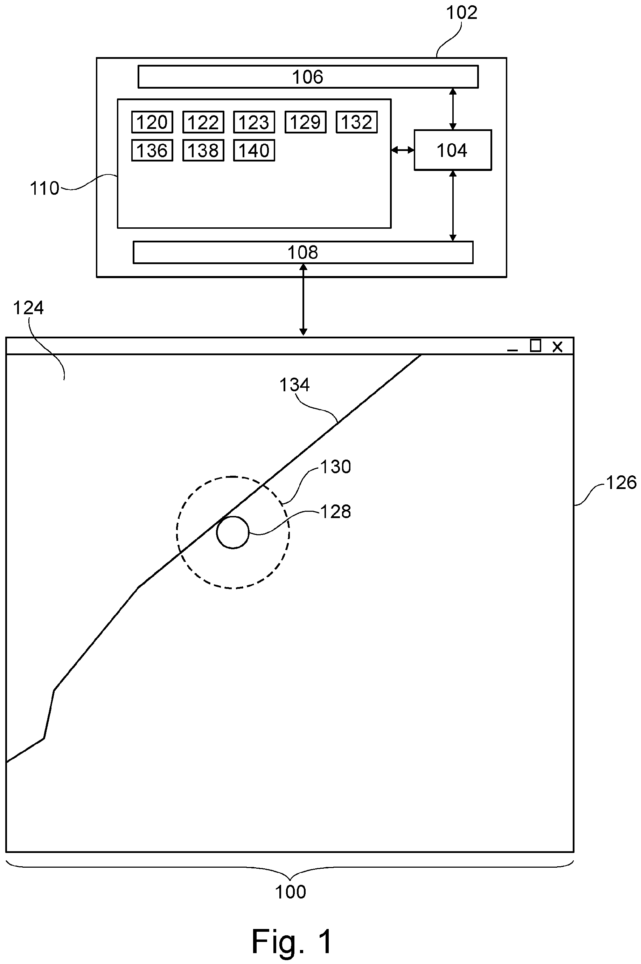

Fig. 1 illustrates an example of a medical system 100. The medical system 100 is shown as comprising a computer 102. The computer 102 may represent one or more computers or computing systems located at one or more locations. The computation 102 could for example be a remote or cloud-based computer. It could also be a workstation in a radiology or other medical facility. The computer 102 may also be part of a medical imaging system or tomographic medical imaging system.

The computer 102 is shown as comprising a computational system 104. The computational system 104 is intended to represent one or more computational systems or computing cores located at one or more locations. The computational system 104 is in communication with an optional hardware interface 106. The hardware interface 106 may enable the computational system 104 to control and/or communicate with other components of the medical system 100 if they are present. The computational system 104 is in further communication with a user interface 108 and a memory 110. The memory 110 is intended to represent various types of memory which may be in communication and be accessible to the computational system 104.

The memory 110 is shown as containing machine-executable instructions 120. The machine-executable instructions 120 enable the computational system 104 to perform basic computing, data, and graphics manipulation tasks. The memory 110 is further shown as containing an image processing neural network 122 that has been received. The image processing neural network 122 may be untrained or it may be already trained or partially trained. The memory is further shows as storing a training three-dimensional medical image 123. This may be a three-dimensional image or a stack of two dimensional images.

The user interface 108 is being used to provide a graphical user interface to a user. The graphical user interface 126 is displaying a cross-sectional view 124 of the training three-dimensional medical image 123. The training three-dimensional medical image 123 is shown as being stored in the memory 110.

On the graphical user interface 126 there is a segmentation entry tool 128 which may be used to manipulate or modify a manual segmentation 132. A rendering of the manual segmentation 132 is displayed on the graphical user interface 126 as a rendering of the manual segmentation 134. In this example the segmentation entry tool 128 is a ball-shaped tool which is used to distort or move the manual segmentation 132. The dashed line 130 surrounding the segmentation entry tool 128 is a predefined distance from the segmentation entry tool 128. As the segmentation entry tool 128 is used, it generates segmentation edit data 129. Voxels within the predefined distance 130 may record per-voxel tool use metadata 136. The per-voxel tool use metadata 136 contains metadata which is descriptive of the use and the control of the segmentation entry tool 128.

For example, things such as velocity or details about the segmentation entry tool 128 such as its size are recorded. As the training three-dimensional medical image 123 is three-dimensional the predefined distance 130 may also affect voxels which are not in the plane of the rendering 124 of the cross-sectional view. For example, the ball-shaped tool 128 may affect the rendering of the manual segmentation 134 for voxels and the segmentation 134 in different planes.

The per-voxel tool use metadata 136 is shown as being stored and recorded in the memory 110. The memory 110 is further shown as containing a per-voxel confidence score 138 for the training three-dimensional medical image 123 using the per-voxel tool use metadata 136. The memory 110 is further shown as containing training data 140 that was constructed from the training three-dimensional medical image 123 and the manual segmentation 132. This is used to train the image processing neural network 122 to produce a manual segmentation when a three-dimensional medical image is input into it.

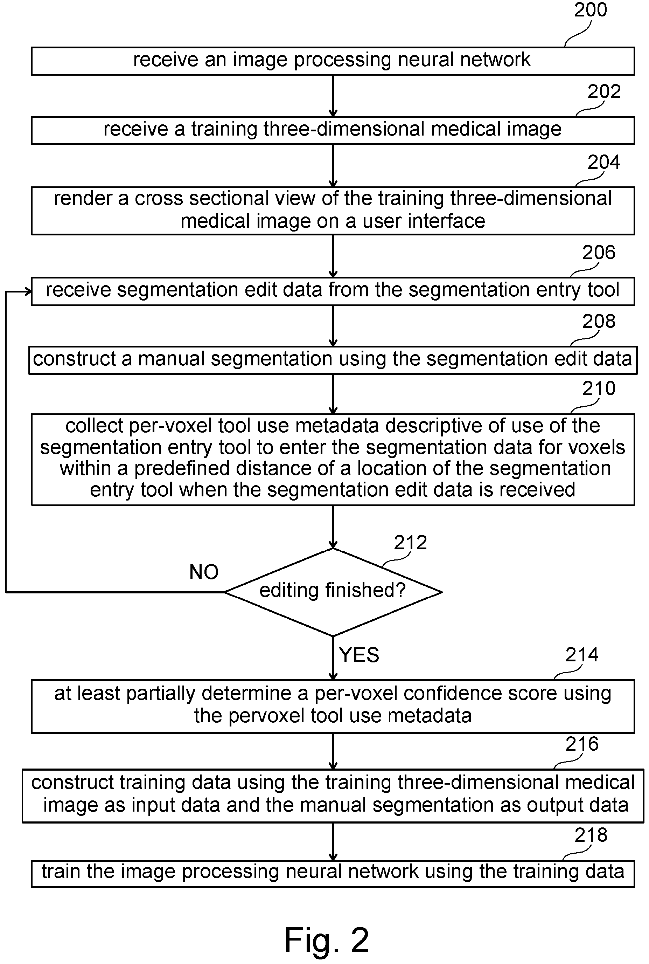

Fig. 2 shows a flowchart which illustrates a method of operating the medical system 100 of Fig. 1. In step 200, the image processing neural network 122 is received. In step 202, the training three-dimensional medical image 123 is received. In step 204 the cross-sectional view 124 of the training three-dimensional medical image 123 is rendered on the user interface 126. In step 206, the segmentation edit data 129 is received from the segmentation entry tool 128. The segmentation edit data 129 is then stored in the memory 110. In step 208, the manual segmentation 132 is constructed or modified using the segmentation edit data 129. In step 210, the per-voxel tool use metadata 136 is collected as the segmentation entry tool 128 is used to generate the segmentation edit data 129. In block 212 there is a question: if the manual segmentation is finished being edited then the method proceeds to block 214. If it is not finished being edited the method proceeds to block 206 where the steps 206, 208, and 210 are repeated until the manual segmentation 132 is complete.

In block 214 the per-voxel confidence score 138 is determined using the per-voxel tool use metadata 136. In block 216 the training data 140 is constructed using the three-dimensional medical image 123 and the manual segmentation 132. In step 218, the image processing neural network 122 is trained using the training data 140. In this example, only one set of training data was constructed. However, in the course of training the image processing neural network 122 this construction of the training data 140 and then training the image processing neural network 122 may be repeated multiple times.

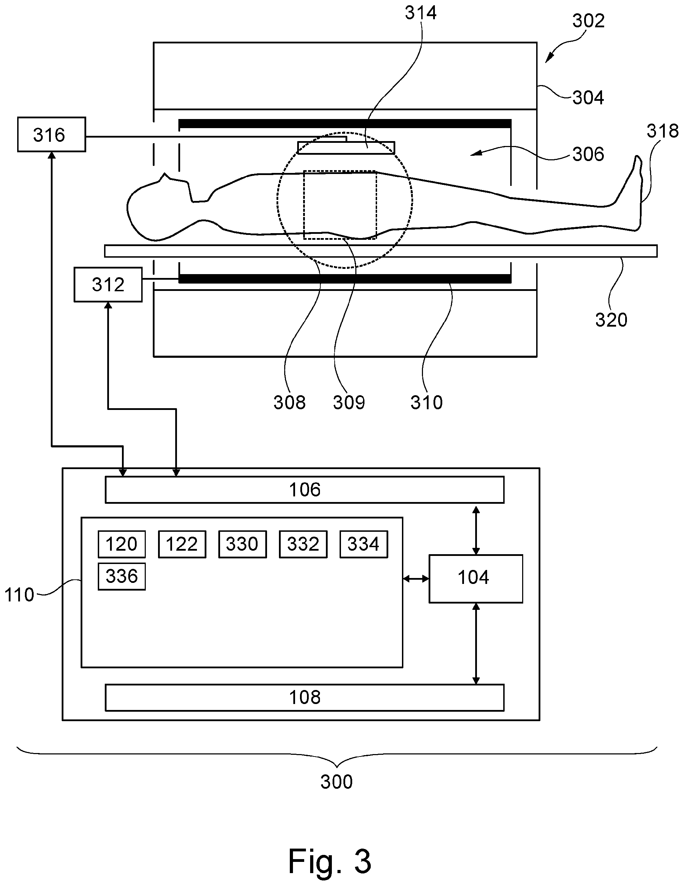

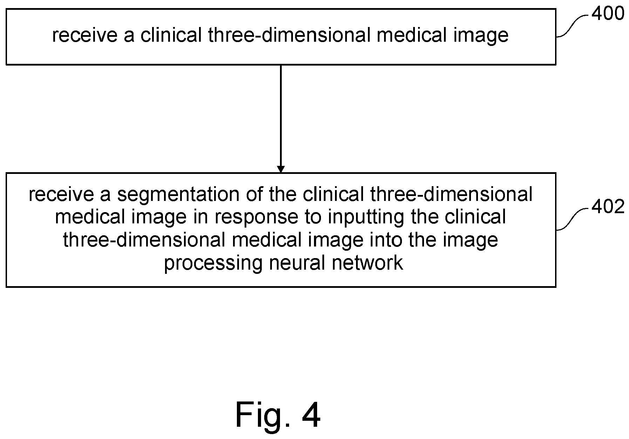

Fig. 3 illustrates a further example of the medical system 300. Fig. 3 illustrates the deployment of the trained image processing neural network 122. The training functionality is illustrated in Figs. 1 and 2 and the use or deployment of the image processing neural network 122 is illustrated in Figs. 3 and 4. However, the features of Figs. 1 and 3 may be combined as well as the features of Figs. 2 and 4 may also be combined.

The medical system 300 illustrated in Fig. 3 is further shown as comprising a magnetic resonance imaging system 302. The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data that is acquired typically acquried for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used for acquisition of preliminary k-space data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels.

The transceiver 316 and the gradient controller 312 are shown as being connected to the hardware interface 106 of the computer system 102.

The memory 110 is again shown as containing the machine-executable instructions 120 and the image processing neural network 122. The memory 110 is shown as additionally containing the pulse sequence commands 330. The pulse sequence command 330 are commands or data which may be converted into commands to control the magnetic resonance imaging system 302 to acquire k-space data 332 according to a three-dimensional medical magnetic resonance imaging protocol. The memory 110 is shown as containing k-space data 332 that has been acquired by controlling the magnetic resonance imaging system 302 with the pulse sequence commands 330. The memory 110 is further shown as containing a clinical three-dimensional medical image 334. In this case the clinical three-dimensional medical image 334 is a magnetic resonance imaging image reconstructed from the k-space data 332. This example has been illustrated using a magnetic resonance imaging system 302, however this is only exemplary. Other medical imaging modalities such as computed tomography, positron emission tomography, single-photon emission tomography as well as three-dimensional ultrasound may also be used. The memory 110 is further shown as containing a segmentation 336 of the clinical three-dimensional medical image 334 that was obtained by inputting the clinical three-dimensional medical image 334 into the image processing neural network 122.

Fig. 4 shows a flowchart which illustrates a method of operating the medical system 300 of Fig. 3. In step 400, the clinical three-dimensional medical image 334 is received. This may include the acquisition of the k-space data 332 as well as its reconstruction of the clinical three-dimensional medical image 334. Step 400 may vary for other imaging modalities. In step 402, the segmentation 336 of the clinical three-dimensional medical image 334 is received in response to inputting the clinical three-dimensional medical image 334 into the image processing neural network 122. The steps illustrated in Fig. 4 may be performed before or after the steps illustrated in Fig. 2.

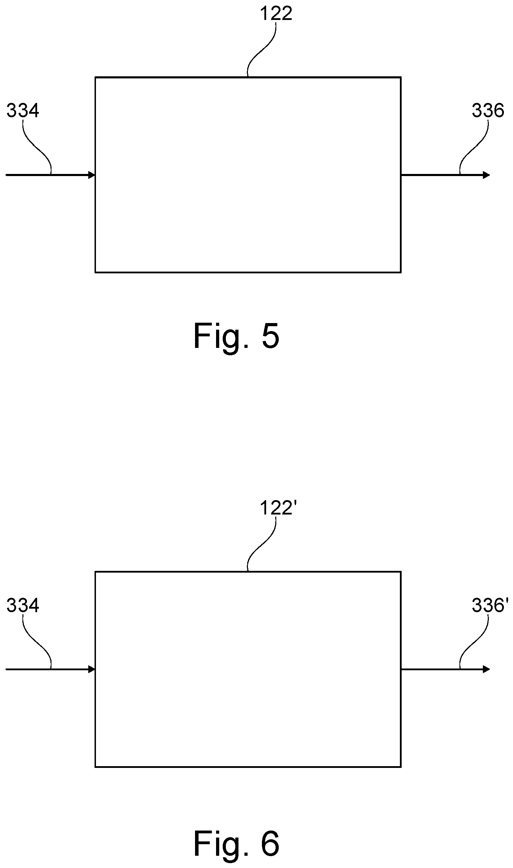

Fig. 5 illustrates an example of the image processing neural network 122. The image processing neural network may for example be a U-net, a ResNet, a convolutional neural network, or other neural network.

The input to the image processing neural network 122 in this example is the clinical three-dimensional medical image 334. It then outputs the segmentation 336 in the form of an identical image that specifies the locations or presence of the segmentation in a voxel or not.

Fig. 6 illustrates an alternative example of the image processing neural network 122'. In this example the image processing neural network 122' again receives the clinical three-dimensional medical image 334 as input, however the segmentation 336' has a different form. Instead of outputting a value for each voxel, the segmentation 336' contains the location of vertices for a three-dimensional mesh which defines the segmentation 336'. The types of neural networks used for outputting this segmentation 336' may be the same as the type of neural networks used for the image processing neural network 122 illustrated in Fig. 5. During the training process the segmentation 336' can be defined by a three-dimensional mesh with a predetermined number of vertices. There is then the coordinates of the individual vertices are output instead of individual values corresponding to individual voxels. The different segmentation 336' in different instances is then simply a distortion of the same segmentation using the same number of vertices.

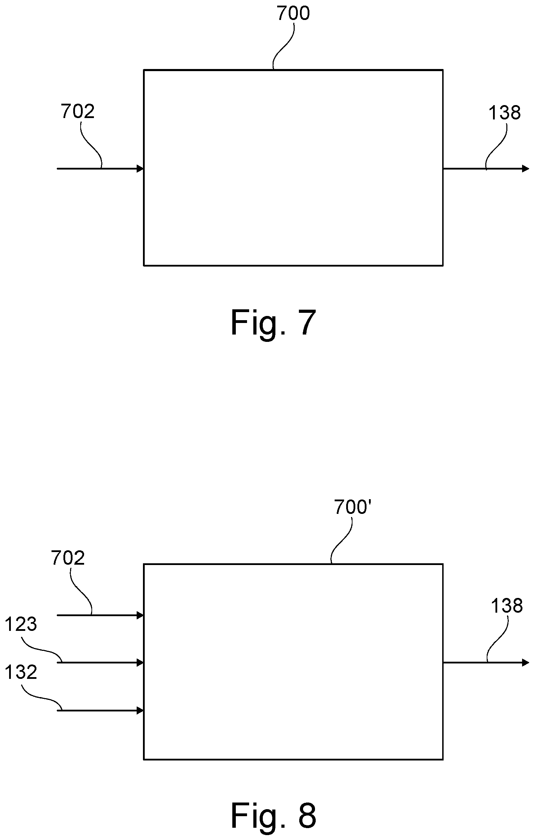

Fig. 7 illustrates an implementation of a trained machine learning module 700. The trained machine learning module 700 may, for example, be a linear regression module, a support vector machine, a random forest, a U-Net, a F-net, or a neural network.

The trained machine learning module 700 is trained to receive the encoded per-voxel tool use metadata 702 and in response output a machine-generated confidence score. In many cases the machine-generated confidence score will directly be the per-voxel confidence score 138.

Fig. 8 illustrates a further example of a trained machine learning module 700'. As input, this trained machine learning module 700' receives the encoded-per-voxel tool use metadata 702 but additionally receives the training three-dimensional medical image 123 and the manual segmentation 132. Then, in response, it outputs the per-voxel confidence score 138 or in some cases, the machine-generated confidence score.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: image processing neural network
- 122': image processing neural network
- 123: training three-dimensional medical image
- 124: rendering of cross sectional view
- 126: graphical user interface
- 128: segmentation entry tool
- 129: segmentation edit data
- 130: predefined distance
- 132: manual segmentation
- 134: rendering of manual segmentation
- 136: per-voxel tool use metadata
- 138: per-voxel confidence score
- 140: training data
- 200: receive an image processing neural network
- 202: receive a training three-dimensional medical image, wherein the training three-dimensional medical image comprises voxels;
- 204: render a cross sectional view of the training three-dimensional medical image on a user interface
- 206: receive segmentation edit data from the segmentation entry tool
- 208: construct a manual segmentation using the segmentation edit data
- 210: collect per-voxel tool use metadata descriptive of use of the segmentation entry tool to enter the segmentation data for voxels within a predefined distance of a location of the segmentation entry tool when the segmentation edit data is received
- 212: editing finished?
- 214: at least partially determine a per-voxel confidence score using the per-voxel tool use metadata
- 216: construct training data using the training three-dimensional medical image as input data and the manual segmentation as output data
- 218: train the image processing neural network using the training data, wherein the training of the image processing data is adjusted using the per-voxel confidence score
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio-frequency coil
- 316: transceiver
- 318: subject
- 320: subject support
- 330: pulse sequence commands
- 332: k-space data
- 334: clinical three-dimensional medical image
- 336: segmentation
- 336': segmentation
- 400: receive a clinical three-dimensional medical image
- 402: receive a segmentation of the clinical three-dimensional medical image in response to inputting the clinical three-dimensional medical image into the image processing neural network
- 700: trained machine learning module
- 700': trained machine learning module
- 702: encoded per-voxel tool use metadata

## Claims

1. A medical system (100, 300) comprising:
- a memory (110) storing machine executable instructions (120);
- a user interface (108); and
- a computational system (104), wherein execution of the machine executable instructions causes the computational system to:
- receive (200) an image processing neural network (122, 122'), wherein the image processing neural network is configured to output a segmentation (336, 336') of at least a portion of a clinical three-dimensional medical image (334) in response to receiving the at least a portion of a clinical three-dimensional medical image as input;
- receive (202) a training three-dimensional medical image (123), wherein the training three-dimensional medical image comprises voxels;
wherein execution of the machine executable instructions further causes the computational system to repeatedly:
- render (204) a cross sectional view (124) of the training three-dimensional medical image on a user interface, wherein the user interface comprises a segmentation entry tool (128);
- receive (206) segmentation edit data (129) from the segmentation entry tool;
- construct (208) a manual segmentation (132) using the segmentation edit data; and
- collect (210) per-voxel tool use metadata descriptive of use of the segmentation entry tool to enter the segmentation edit data for voxels within a predefined distance (130) of a location of the segmentation entry tool when the segmentation edit data is received;
wherein execution of the machine executable instructions further causes the computational system to: -
at least partially determine (214) a per-voxel confidence score (138) using the per-voxel tool use metadata;
- construct (216) training data (140) using the training three-dimensional medical image as input data and the manual segmentation as output data; and
- train (218) the image processing neural network using the training data, wherein the training of the image processing neural network is adjusted using the per-voxel confidence score.

2. The medical system of claim 1, wherein the per-voxel tool use metadata comprises a zoom factor of the rendering of the cross-sectional view during entry of the segmentation edit data.

3. The medical system of claim 1 or 2, wherein the per-voxel tool use metadata comprises a description if per-voxel tool use metadata for a voxel was rendered in the user interface when the segmentation edit data was entered.

4. The medical system of claim 1, 2, or 3, wherein the per-voxel tool use metadata comprises a segmentation entry tool type.

5. The medical system of any one of any one of the preceding claims, wherein the per-voxel tool use metadata comprises any one of the following: a segmentation entry tool size, a ball size, a live-wire diameter, a point annotation size, and an interactive region growing size.

6. The medical system of any one of any one of the preceding claims, wherein the per-voxel tool use metadata comprises any one of the following: a maximum segmentation entry tool velocity, a minimum segmentation entry tool velocity, an average segmentation entry tool velocity, and combinations thereof.

7. The medical system of any one of any one of the preceding claims, wherein the per-voxel tool use metadata comprises any one of the following: a flag indicating eraser tool use within the predefined distance, a counter indicating a number of revisions to the manual segmentation the within a predefined distance, and combinations thereof.

8. The medical system of any one of the preceding claims, wherein the per-voxel confidence score is a metric calculated using the per-voxel tool use metadata.

9. The medical system of any one of the preceding claims, wherein the memory further stores a trained machine learning module (700, 700') configured to output a machine generated confidence score for the voxels in response to receiving the per-voxel tool use metadata as input; wherein execution of the machine executable instructions further comprises receiving the machine generated confidence score for the voxels in response to inputting the per-voxel tool use metadata as input into the trained machine learning module.

10. The medical system of claim 9, wherein the trained machine learning module is further configured to receive the training three-dimensional medical image and the manual segmentation as input with the per-voxel tool use metadata, and wherein execution of the machine executable instructions further causes the computational system to input the training three-dimensional medical image and the manual segmentation as input with the per-voxel tool use metadata into the trained machine learning module.

11. The medical system of any one of the preceding claims, wherein the image processing neural network is trained using a loss function, wherein the training of the image processing data is adjusted using the per-voxel confidence score by weighting a contribution by individual voxels to loss function using the per-voxel confidence score.

12. The medical system of any one of the preceding claims, wherein the image processing neural network is configured to receive only a windowed portion of the training three-dimensional image, wherein the training of the image processing neural network is adjusted using the per-voxel confidence score by restricting the windowed portion of the training three-dimensional image to regions where the per-voxel confidence score is above a predetermined threshold.

13. The medical system of any one of the preceding claims, wherein execution of the machine executable instructions further causes the computational system to:
- receive (400) a clinical three-dimensional medical image (334); and
- receive (402) a segmentation (336, 336') of the clinical three-dimensional medical image in response to inputting the clinical three-dimensional medical image into the image processing neural network.

14. A medical imaging method, wherein the method comprises:
- receiving (200) an image processing neural network (122, 122'), wherein the image processing neural network is configured to output a segmentation (336, 336') of at least a portion of a clinical three-dimensional medical image (334) in response to receiving the at least a portion of a clinical three-dimensional medical image as input;
- receiving (202) a training three-dimensional medical image (123), wherein the training three-dimensional medical image comprises voxels;
wherein the method comprises repeatedly:
- rendering (204) a cross sectional view (124) of the training three-dimensional medical image on a user interface, wherein the user interface comprises a segmentation entry tool (128);
- receiving (206) segmentation edit data (129) from the segmentation entry tool;
- constructing (208) a manual segmentation (132) using the segmentation edit data; and
- collecting (212) per-voxel tool use metadata descriptive of use of the segmentation entry tool to enter the segmentation edit data for voxels within a predefined distance (130) of a location of the segmentation entry tool when the segmentation edit data is received;
wherein the method further comprises:
- at least partially determining (214) a per-voxel confidence score (138) using the per-voxel tool use metadata;
- constructing (216) training data (140) using the training three-dimensional medical image as input data and the manual segmentation as output data; and
- training (218) the image processing neural network using the training data, wherein the training of the image processing neural network is adjusted using the per-voxel confidence score.

15. A computer program comprising machine executable instructions (120) configured for execution by a computational system controlling a medical system, wherein the medical system comprises a user interface (108), wherein execution of the machine executable instructions causes the computational system to:
- receive (200) an image processing neural network (122, 122'), wherein the image processing neural network is configured to output a segmentation of at least a portion of a clinical three-dimensional medical image (334) in response to receiving the at least a portion of a clinical three-dimensional medical image as input;
- receive (202) a training three-dimensional medical image (123), wherein the training three-dimensional medical image comprises voxels;
wherein execution of the machine executable instructions further causes the computational system to repeatedly:
- render (204) a cross sectional view (124) of the training three-dimensional medical image on a user interface, wherein the user interface comprises a segmentation entry tool (128);
- receive (206) segmentation edit data (129) from the segmentation entry tool;
- construct (208) a manual segmentation (132) using the segmentation edit data; and
- collect (212) per-voxel tool use metadata descriptive of use of the segmentation entry tool to enter the segmentation edit data for voxels within a predefined distance (130) of a location of the segmentation entry tool when the segmentation edit data is received; and
wherein execution of the machine executable instructions further causes the computational system to: -
at least partially determine (214) a per-voxel confidence score (138) using the per-voxel tool use metadata;
- construct (216) training data (140) using the training three-dimensional medical image as input data and the manual segmentation as output data; and
- train (218) the image processing neural network using the training data, wherein the training of the image processing neural network is adjusted using the per-voxel confidence score.
